# EUROPEAN PATENT SPECIFICATION

(11) **EP 2 185 189 B1**
(45) Date of publication and mention of the grant of the patent: **27.09.2017**
(21) Application number: 08794740.4
(22) Date of filing: 25.07.2008
(51) Int. Cl.: A61K 39/02

(54) **CAPSULE COMPOSITION FOR USE AS IMMUNOGEN AGAINST CAMPYLOBACTER JEJUNI**
KAPSELZUSAMMENSETZUNG ZUR VERWENDUNG ALS IMMUNOGEN GEGEN CAMPYLOBACTER JEJUNI
COMPOSITION DE CAPSULE POUR UNE UTILISATION EN TANT QU'IMMUNOGÈNE CONTRE CAMPYLOBACTER JEJUNI

(30) Priority: 27.07.2007 US 962313 P
(43) Date of publication of application: 19.05.2010
(60) Divisional application: 14001557.9
(73) Proprietor: The United States Of America As Represented By The Secretary Of The Navy, Silver Spring, MD 20910 (US); UNIVERSITY OF GUELPH, Guelph, Ontario N1G 5G3 (CA)
(72) Inventor: GUERRY-KOPECKO, Patricia, Silver Spring, MD 20906 (US); MONTEIRO, Mario, Artur, Guelph, Ontario N1E 7A5 (CA)
(74) Representative: Grosse Schumacher Knauer von Hirschhausen
(86) International application number: PCT/US2008/009032
(87) International publication number: WO 2009/017666

(56) References cited:
- WO-A1-2005/020964
- WO-A2-2007/038122
- US-A- 5 874 300
- US-A1- 2005 123 538
- US-A1- 2005 136 435
- University of Vermont: "Campylobacter Jejuni Challenge Model Development: Dose Ranging Study", ClinicalTrials.gov Clinical Trials, no. NCT00434798 1 March 2007 (2007-03-01), XP55002090, Retrieved from the Internet: URL:http://dfpcorec-p/wf/web/citenpl/citen pl.html [retrieved on 2011-07-06]
- CHEN ET AL: "The chemical structure and genetic locus of Campylobacter jejuni CG8486 (serotype HS:4) capsular polysaccharide: the identification of 6-deoxy-d-ido-heptopyranose", CARBOHYDRATE RESEARCH, PERGAMON, GB, vol. 343, no. 6, 4 March 2008 (2008-03-04) , pages 1034-1040, XP022576072, ISSN: 0008-6215, DOI: DOI:10.1016/J.CARRES.2008.02.024
- PAPP-SZABO ET AL: "Cell-surface alpha-glucan in Campylobacter jejuni 81-176", CARBOHYDRATE RESEARCH, PERGAMON, GB, vol. 340, no. 13, 26 September 2005 (2005-09-26), pages 2218-2221, XP005032116, ISSN: 0008-6215, DOI: DOI:10.1016/J.CARRES.2005.06.023
- ASPINALL G O ET AL: "Structures of the O chains from lipopolysaccharides of Campylobacter jejuni serotypes O:23 and O:36", CARBOHYDRATE RESEARCH, PERGAMON, GB, vol. 231, 2 July 1992 (1992-07-02), pages 13-30, XP026634721, ISSN: 0008-6215, DOI: DOI:10.1016/0008-6215(92)84003-B [retrieved on 1992-07-02]
- ANDREY V KARLYSHEV ET AL: "Analysis of Campylobacter jejuni Capsular Loci Reveals Multiple Mechanisms for the Generation of Structural Diversity and the Ability to Form Complex Heptoses", MOLECULAR MICROBIOLOGY, WILEY-BLACKWELL PUBLISHING LTD, GB, vol. 55, no. 1, 1 January 2005 (2005-01-01), pages 90-103, XP008126621, ISSN: 0950-382X, DOI: DOI:10.1111/J.1365-2958.2004.04374.X

## Description

### FIELD OF INVENTION

The inventive subject matter relates to an immunogenic composition capable of conferring protection against diarrhea caused by *Campylobacter jejuni* and a method of inducing an immune response to said composition.

### BACKGROUND OF INVENTION

*C*. *jejuni* is a leading cause of diarrheal disease worldwide and a documented threat to US military personnel (Taylor, 1992; Tauxe, 1992). The symptoms of campylobacter enteritis include diarrhea, abdominal pain, and fever and often accompanied by vomiting. Stools usually contain mucus, fecal leukocytes, and blood, although watery diarrhea is also observed (Cover and Blaser 1999). However, despite the importance of this organism to human disease, there are no licensed vaccines against *C. jejuni.*

Because of the medical importance of *C*. *jejuni,* considerable research is dedicated toward understanding the pathogen. However, notwithstanding this effort, there is surprisingly little understanding about how *C. jejuni* causes human disease.

University of Vermont: "Campylobacter Jejuni Challenge Model Development: Dose Ranging Study", 1 March 2007 describes human immune responses to whole cell *Campylobacter jejuni* infection.

The genome of one strain, NCTC 11168 (Parkhill, et al., 2000) revealed several unusual aspects about the biology of *C. jejuni.* One striking feature is the presence of an unexpectedly high number of genes encoding putative enzymes involved in sugar and/or polysaccharide synthesis (Parkhill et al., 2000). The sequence, and resulting research fostered primarily by the availability of the sequence, has revealed that these genes fall into 4 main functional clusters that underscore the importance of some unusual carbohydrate structure to the biology of *C. jejuni.* These clusters include Lipooligosaccharide (LOS) synthesis, genetic control of flagellin glycosylation, genetic control of N-linked glycosylation, and the control of the biosynthesis and assembly of capsule.

Vaccine strategies against *C*. *jejuni* have been largely limited due to the molecular mimicry between lipooligosaccharide (LOS) cores of many strains of *C*. *jejuni* and human gangliosides (Moran, et al., 1996). This mimicry is thought to be a major factor in the strong association of *C*. *jejuni* infection with Guillain Barre Syndrome (GBS), a post-infectious polyneuropathy (Allos, 1997). Thus, antibodies generated against LOS cores result in an autoimmune response to human neural tissue. It has been estimated that as many as 1/3000 cases of campylobacter enteritis results in GBS. Therefore, the possibility of developing GBS could be associated with any whole cell vaccine against *C*. *jejuni* that includes ganglioside mimicry.

LOS synthesis in Campylobacter is controlled by a number of genes, including genes encoding enzymes involved in biosynthesis of sialic acid for incorporation into LOS. Thus, *C. jejuni* is one of a limited number of bacteria that can endogenously synthesize sialic acid, a 9 carbon sugar that is found in many mammalian cells. This is consistent with the observed molecular mimicry of LOS and human gangliosides important in GBS (Aspinall et al., 1993, 1994 (a and b); Salloway et al., 1996).

Although glycosylation of proteins was once considered to be a eukaryotic trait, there is an increase awareness of prokaryotic protein glycosylation (Power and Jennings, 2003). The best characterized and most extensively glycosylated bacterial protein is campylobacter flagellin. Flagellin from strain 81-176 is glycosylated at 19 serine or threonine sites by an O-linkage to pseudaminic acid and derivatives of pseudaminic acid (Thibault et al., 2001). Pseudaminic acid is an unusual 9 carbon sugar that resembles sialic acid, but which is highly immunogenic, unlike sialic acid. Moreover, mutants that are unable to glycosylate flagellin cannot assemble a flagellar filament (Goon et al, 2003). Since flagella are indispensable virulence determinants of *C*. *jejuni,* glycosylation is therefore also a key virulence determinant.

One of the most unusual aspects of *C. jejuni* is the presence of a general system for N-linked glycosylation of numerous proteins (Szymanski et al., 1999; reviewed in Szymanski et al., 2003). This system, which includes an oligosaccharide transferase similar to that found in the eukaryote *Saccharomyces cerevisiae,* attaches a glycan which has recently been shown to be a heptasaccharide composed of one bacillosamine residue (an unusual deoxy sugar), one D-glucose, and five D-GaINAc residues (Young et al., 2002). The glycosylation appears to occur on numerous periplasmic, and perhaps, surface exposed proteins in *C*. *jejuni* (Young et al., 2002). The unusual glycan, again, appears to be highly immunogenic and is recognized during human infection (Szymanski et al., 1999, 2003).

An interesting recent revelation regarding the Campylobacter genome sequence was the presence of a complete set of capsule transport genes similar to those seen in type II/III capsule loci in the *Enterobactericeae* (Parkhill et al., 2000; Karlyshev et al., 2000). Subsequent genetic studies in which site-specific mutations were made in several capsule transport genes indicated that the capsule was the serodeterminant of the Penner serotyping scheme (Karlyshev et al., 2000; Bacon et al., 2001). The Penner scheme (or HS for heat stable) is one of two major serotyping schemes of campylobacters and was originally thought to be based on lipopolysaccharide O side chains (Moran and Penner, 1999).

Currently it is believed that all of the structures previously described as O side chains are, in fact, capsules. The chemical structures of the capsule/O side chains of several Penner serotypes have been determined, and these structures include several unusual sugar structures, as summarized in Table 1. The capsule of the genome strain, NCTC 11168, contains a heptopyranose as a L*-gluco* conformer, which is the first report of such a structure in nature (St. Michael et al., 2002). The capsule of the type strains HS23 and HS36 contain the same carbohydrates in different ratios, and include a mixture of 4 unusual *altro*-heptoses (6-deoxy-α-D-*altro*-heptose, D-glycero-α-D-*altro*-heptose, 6-deoxy-3-Me-α-D-*altro*-heptose, and 3-Me-D-glycero-α-D-*altro*-heptose (Aspinall et al., 1992).

**Table 1. Structure of some capsular polysaccharides of C. jejuni strains.**

| **Strain** | **Structure** | **Reference** |
|---|---|---|
| HS3 | →4-α-D-Gal-(1→3)(3-hydroxypropanoyl)-L-glycero-α-D-ido-Hep-(1→ | Aspinall et al., 1995 |
| HS19 | →4)-β-D-GlcA-(1→3)-β-D-GlcNAc-(1→ | Aspinall et al., 1994 a, b |
| | (the GlcA units are present as amides of 2-amino-2-deoxyglycerol) | |
| HS23, HS36 | Four closely-related polysaccharides: | Aspinall et al., 1992 |
| | →3)-β-D-GlcNAc-(1→3)-α-D-Gal-(1→2)-6d-α-D-altro-Hep-(1→; | |
| | →3)-β-D-GlcNAc-(1→3)-α-D-Gal-(1→2)-6d-3-O-Me-α-D-altro-Hep-(1→; | |
| | →3)-β-D-GlcNAc-(1→3)-α-D-Gal-(1→2)-D-glycero-α-D-altro-Hep-(1→; | |
| | →3)-β-D-GlcNAc-(1→3)-α-D-Gal-(1→2)-3-O-Me-D-glycero-α-D-altro-Hep-(1→; | |
| 81116 | Two polysaccharides at a ratio of 3A : 1B, where | Muldoon et al. (2002) |
| | 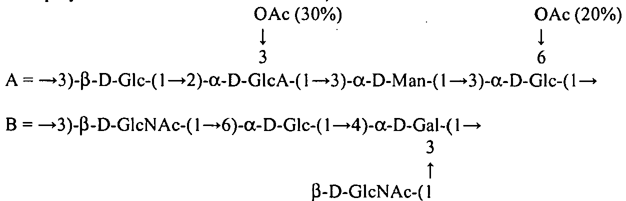 | |
| NCTC 11168 | 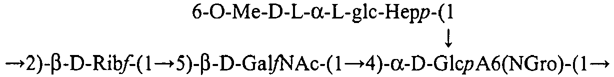 | St. Michael et al. (2002) |
| | (Here, Glucuronic acid is amidated with 2-amino-2-deoxyglycerol at C-6) | |

There are several examples of highly effective capsular vaccines. *S*. *pneumoniae* has 83 different capsular types, but the current *S. pneumoniae* vaccine contains a mixture of the 23 most prevalent serotypes in the US and Europe. *N*. *meningiditis* has fewer serogroups, thus potentially simplifying vaccine development, and, in fact, serogroups A, B and C are responsible for >90% of cases of meningococcal meningitis (Jennings, 1990). However, the polysaccharide from serotype B is poorly immunogenic in man, likely because it mimics human tissues. Capsular vaccines have also been developed against *H. influenzae* and Group B *Streptococcus.*

As previously mentioned, there currently are no licensed vaccines against Campylobacter, due greatly to the molecular mimicry between LOS cores of many strains of *C*. *jejuni* and human gangliosides (Moran, et al., 1996). However, vaccine formulations incorporating bacterial capsules have been developed against a number of pathogens. In general, capsule vaccines are immunogenic in humans and nontoxic (Jennings, 1990). One of the general problems associated with capsule vaccines is the poor immunogenicity of all polysaccharides in infants, and the fact that many of the capsular vaccines are directed at diseases that are particular threatening to the pediatric population. Based on murine studies, pure polysaccharide antigens are considered to be T cell independent, and capable of inducing only IgM type responses. Adult humans, in contrast, are able to generate IgG, in addition to IgM and IgA antibodies against polysaccharides. Responses in infants to vaccines against type B *H. influenzae* (Schneerson et al 1980; Anderson, 1983; Marburg, 1986), group A, B and C *Neisseria meningiditis* (Jennings and Lugowski, 1981 and 1983; and type 6A *Streptococcus pneumoniae* (Chu et al., 1983) have all improved following conjugation to proteins.

*C*. *jejuni* capsule, as defined in this application, is a generic term for capsular polymers, which are composed of repeating polysaccharide structures. The repeating structures can be homopolymers, defined as a repeating single sugar moiety, or repeating oligosaccharides (i.e. disaccharides or trisaccharides, etc.). A number of species of capsular repeating polysaccharide polymers have been identified. To illustrate the genus of capsular polysaccharide structures, Table 2 lists known capsular polysaccharide structures for Campylobacter strains.

**Table 2**

| **Strain/HS type** | **Structure** | **Reference** |
|---|---|---|
| HS3 | →4-α-D-Gal-(1→3)-(3-hydroxypropanol)-L-glycero-α-D-ido-Hep-(1→ | Aspinall, et al (1995 |
| HS19 | →4)-β-D-GlcA-(1→3)-β-D-GlcNac-(1→ (GlcA units are present as amides of 2-amino-2-deoxyglycerol) | Aspinall, et al., 1994 (a and b) |
| HS23/36 | Four closely-related polysaccharides: | Aspinall, et al., 1992 |
| | →3)-β-D-GlcNAc-(1→3)-α-D-Gal-(1→2)-6d-α-D-altro-Hep-(1→; | |
| | →3)-β-D-GlcNAc-(1→3)-α-D-Gal-(1→2)-6d-3-O-Me-α-D-altro-Hep-(1→; | |
| | →3)-β-D-GlcNAc-(1→3)-α-D-Gal-(1→2)-D-glycero-α-D-altro-Hep-(1→; | |
| | →3)-β-D-GlcNAc-(1→3)-α-D-Gal-(1→2)-3-O-Me-glycero-α-D-altro-Hep-(1→; | |
| 81116 (HS6) | Two polysaccharides at a ratio of 3A:1B, where: | Muldoon, et al., 2002 |
| | 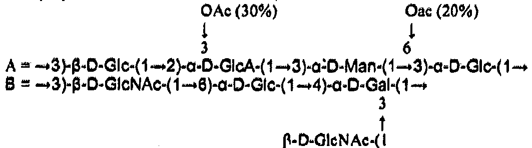 | |
| NCTC 11168(HS2) | 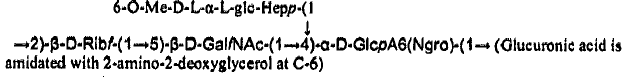 | St. Michael, et al., 2002 |
| HS41 | Two closely related polysaccharides: | Hannify, et al., 1999 |
| | →2)-β-L-Ara*f*-(1→2)-β-D-6d-altro-Hep*f*-(1→2)-β-L-6d-altro*f*-(1→ (75%; and | |
| | →2)-β-L-Ar*f*(1→2)-β-D-6d-altro-Hep*f*-(1→2)-α-D-Fuc*f*-(1→; (25%) | |
| HS30 (C. coli) | 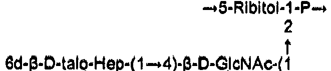 | Aspinall, et al., 1993 |
| HS1 | →4)-β-D-Gal-(1→2)-(*R*)-Gro-(1-P→ | Aspinall 1998; McNally, et al., 2005 |
| | (with two branches at C-2 and C-3 of Gal of β-D fructofuranoses that are further substituted at C-3 with O-methyl phosphoramidate groups | |
| HS 4 | →3)-6-d-β-D-ido-Hep-(1→4)-β-D-GlcNac-(1→ | Chen, et al. 2008 |
| | With O-methyl phosphoramidate units present in non-stoichiometric amounts at the 0-2 and/or 0-7 positions of 6-deoxy-beta-D-ido-Heptose. | |
| 81-176 (US23/36) | 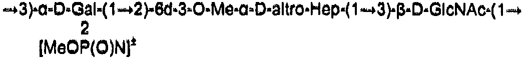 | Kanipes, et al., 2006 |

WO 2007/038122 describes an immunogenic composition which is composed of *Campylobacter jejuni* capsule polysaccharide polymer with a repeating disaccharide structure →3)-α-6-deoxy-D-ido-Heptose (I→4)-β-D-GlcNAc-(I→.

### SUMMARY OF INVENTION

In one aspect the invention provides ann immunogenic composition, composed of an isolated polymer, wherein said polymer contains repeat units of a disaccharide having the formula →4)[P-→3]-a pha-D-Ga-(1→3)-[P→2/7]-6d-alpha-D-ido-Hep-(1→, wherein P is non-stoichiometic O-methyl phosphoramidate.

In one embodiment said repeating disaccharide is conjugated to a carrier molecule.

In another embodiment said isolated polymer further comprises 3-hydroxypropanoyl.

In yet another embodiment said isolated polymer contains 2-100 repeat units of said disaccharide.

In another aspect the invention provides for the composition described above for use in the prevention of Campylobacter jejuni infections.

In some embodiments said isolated polymer is conjugated to a carrier molecule.

In a apreferred embodiment the carrier molecule is CRM197.

in another embodiment said immunogenic composition contains 2-100 repeat units said disaccharide.

### DESCRIPTION OF DRAWINGS

**Figure 1****.** Sugar composition analysis of the capsular polysaccharide of *C*. *jejuni* strain BH01-0142 (hereafter referred to as BH0142) showing that this capsular polysaccharide is composed in part of D-galactose, 6-deoxy-D-ido-heptose, and L-glycero-D-ido-heptose.
**Figure 2****.** (A) ¹H-NMR spectrum of the capsular polysaccharide of *C. jejuni* strain BH0142 showing that this capsular polysaccharide, and (B) ³¹PNMR spectrum of the capsular polysaccharide of *C*. *jejuni* strain BH0142 showing that this capsular polysaccharide contains several O-methyl-phosphoramidate units.
**Figure 3****.** The chemical structure of the disaccharide repeating blocks that make up the capsular polysaccharide of *C*. *jejuni* strain BH0142.
**Figure 4****.** Sugar composition analysis of the activated (oxidized) capsular polysaccharide of *C*. *jejuni* strain BH0142 showing that the activated capsular polysaccharide is composed in part of idose (with an aldehyde at C-6), 6-deoxy-D-ido-heptose, and L-glycero-D-ido-heptose.
**Figure 5****.** (A) Scheme showing the conjugation of the activated *C. jejuni* strain BH0142 capsular polysaccharide to the carrier protein CRM₁₉₇, and (B) Scheme showing the conjugation of the activated *C*. *jejuni* strain CPS8486 capsular polysaccharide to the carrier protein CRM₁₉₇. Either a non-reducing end GlcNAc or a non-reducing end 6d-ido-Hep could be oxidized.

### DETAILED DESCRIPTION OF PREFERRED EMBODIMENTS

*C. jejuni* capsular moieties are important in serodetermination. However, despite over 60 Penner serotypes having been identified, most Campylobacter diarrheal disease is caused by *C. jejuni* from a limited number of serotypes. Because of the importance of capsule structure in serodetermination, it is postulated that they are highly immunogenic structures. Additionally, they are also unlikely to exhibit the unwanted autoimmune induction caused by immuno-mimicry observed by lipooligosaccharides. Therefore, capsules or capsular components would be highly useful in anti- *C. jejuni* vaccines. *C. jejuni* capsule are composed of repeating polysaccharide structures. The repeating structures can be homopolymers, defined as a repeating single sugar moiety, or repeating oligosaccharides such as disaccharides or trisaccharides.

The chemical composition of *C*. *jejuni* capsules were analyzed by first growing *C. jejuni* and then isolating and purifying the capsule using water-phenol extraction, ultra-centrifugation and gel permeation chromatography. The specific carbohydrate structures were determined by chemical manipulations in combination with gas-liquid chromatography (GLC), and GLC-mass spectrometry, and fast atom bombardment-mass spectrometry (FAB-MS). Anomeric configuration of the sugars was determined by nuclear magnetic resonance (NMR) spectrometry.

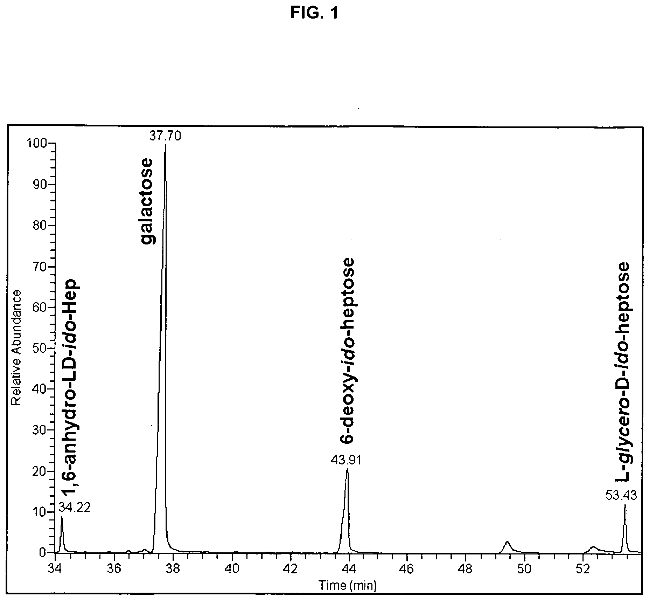

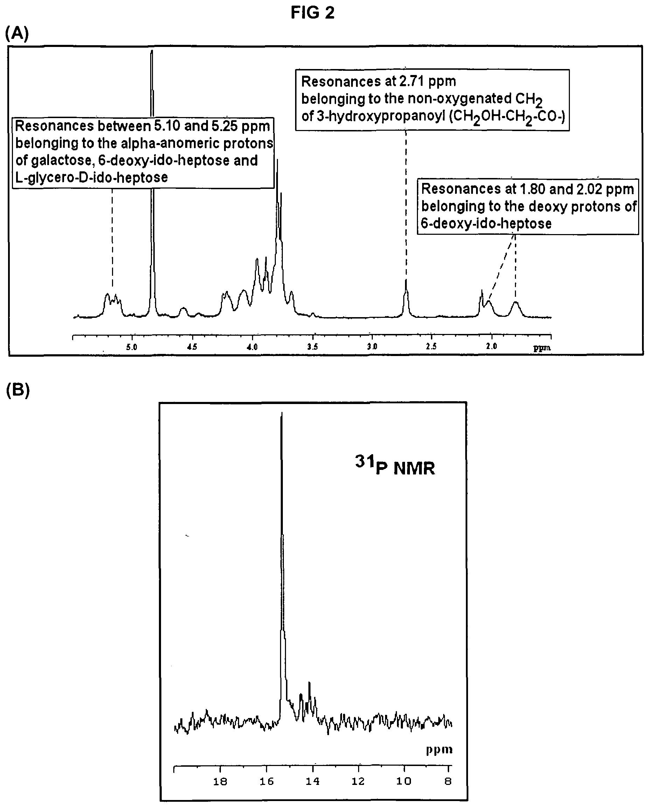

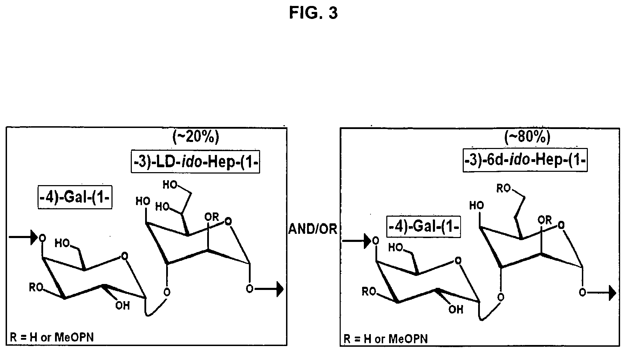

Based on carbohydrate analyses as shown in FIG. 1, the capsule of *C. jejuni* strain BH0142 (a representative of H3 serotype complex) was composed of D-galactose and 6-deoxy-D-ido-heptose, and with smaller amounts of L-glycero-D-ido-heptose. 6-anhydro-L-glycero-D-ido-heptose, a product arising from cyclization of L-glycero-D-ido-heptose during the step1 of hydrolysis, was also observed in this analysis. Sugar linkage-type analysis showed that main monosaccharide units present were 4-substituted D-galactose and 3-substituted 6-deoxy-D-ido-heptose, and smaller amounts of 3-substituted L-glycero-D-ido-heptose also present. The ¹H-NMR spectrum revealed that all units contained the alpha anomeric configuration (Figure 2A). The ¹H-NMR spectrum (Figure 2A), and a 2D ¹H-¹³C HSQC experiment also yielded resonances at δ_{H} 2.71 and δ_{C} 37.20 characteristic of a non-oxygenated methylene of a 3-hydroxypropanoyl unit (7), which revealed the presence of a 3-hydroxypropanoyl (CH₂OH-CH₂-CO- moiety in the capsule polysaccharide of *C. jejuni* strain BH0142. The capsular polysaccharide also contains in part O-methyl phosphoramidate units in variable concentrations (Figure 2B). Linkage analysis data showing 3,4-disubstituted galactose, 2,3-disubstituted 6-deoxy-D-ido-heptose, 3,7-disubstituted 6-deoxy-D-ido-heptose, and 2,3-disubstituted L-glycero-D-ido-heptose, suggest that the O-methyl phosphoramidate units are present at the O-3 position of galactose, at the O-2 and/or O-7 positions of 6-deoxy-alpha-D-ido-heptose, and at the O-3 position of L-glycero-D-ido-heptose. Moreover, the linkage-type analyses show that O-methyl-phosphoramidate is mainly present at the O-3 position of galactose and at the O-2 position of 6-deoxy-alpha-D-ido-heptose. Collectively, the data showed that *C. jejuni* strain BH0142 capsular polysaccharide (Figure 3) was composed of a disaccharide repeating unit composed of 4-substituted alpha-D-galactose and 3-substituted 6-deoxy-alpha-D-ido-heptose. Some disaccharide repeating units (approximately 20%) contained 3-substituted L-glycero-alpha-D-ido-heptose in place of 3-substituted 6-deoxy-alpha-D-ido-heptose. Thus, the disaccharide repeating unit of the capsular polysaccharide of *C*. *jejuni* strain BH0142 has the general structure (Figure 3):

→4)-[P→3]-alpha-D-Gal-(1→3)-[P→2/7]-6-d-alpha-D-ido-Hep-(1→

or

→4)-[P→3]-alpha-D-Gal-(1→3)-[P→2/7]-L-glycero-alpha-D-ido-Hep-(1→

where P represents O-methyl-phosphoramidate and is present in non-stoichiometric amounts. In some disaccharides, 3-hydroxypropanoyl may also be present.

Therefore, an immunogenic formulation composed of isolated capsular polysaccharide composed of disaccharide repeats, each disaccharide having general formula:

→4)-[P→3]-alpha-D-Gal-(1→3)-[P→2/7]-6d-alpha-D-ido-Hep-(1→

or

→4)-[P→3]-alpha-D-Gal-(1→3)-[P→2/7]-L-glycero-alpha-D-ido-Hep-(1→,

where P represents O-methyl-phosphoramidate and is present in non-stoichiometric amounts is described herein. Alternatively the formulation can be composed of a capsular polysaccharide containing a mixture of both disaccharide structures. MALDI-TOF-MS analyses revealed that the average molecular weight of the BH0142 CPS analyzed here was approximately 8300 Da.

### Example 1: Immunity to capsule - can be increase by conjugation to carrier molecules

Since IgO response is often predominantly observed as a T-cell independent immune response. Therefore, children are typically only capable of mounting an IgM response in the face of polysaccharide antigens with adults capable of generating an IgG, IgA and IgM response.

In order to potentially further improve the response to capsule moieties, the immunogenicity of *C*. *jejuni* capsule can be conjugated to T-dependent carrier proteins.

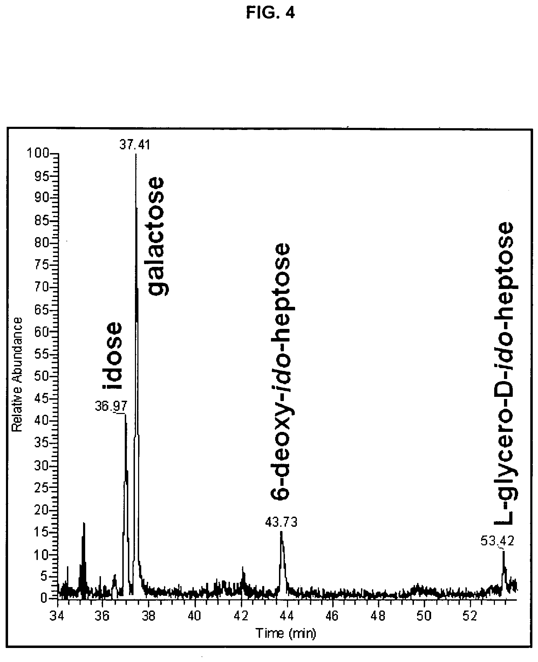

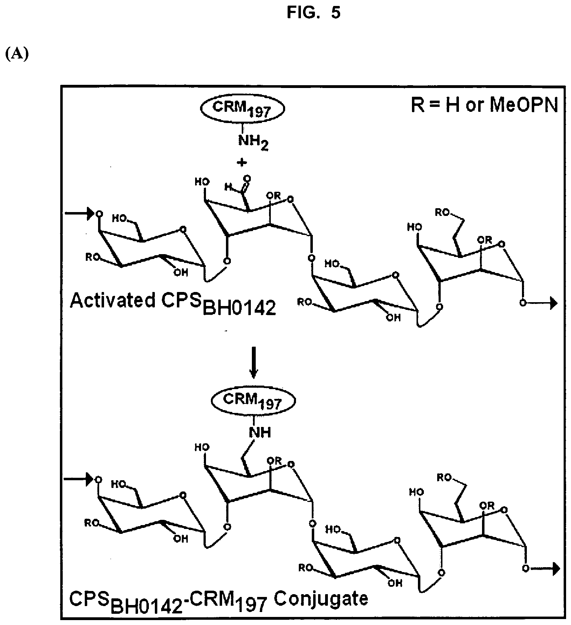

Conjugation of *C*. *jejuni* strain BH0142 capsular polysaccharide to a carrier protein, such as cross reacting material 197 (CRM197), can be achieved by selectively oxidizing the exocyclic glycero moiety, with periodate, of one or more L-glycero-D-ido-heptose units present in each capsular polysaccharide. Analysis (Figure 4) of the activated (oxidized) *C. jejuni* strain BH0142 capsular polysaccharide revealed that indeed L-glycero-D-ido-heptose could be selectively oxidized to an idose unit containing an aldehyde group at C-6, with the remainder of the capsular polysaccharide intact. This activated capsular polysaccharide can be directly coupled to a carrier protein (Figure 5A) by a reductive amination mechanism to yield a glycoconjugate composed of *C*. *jejuni* strain BH0142 capsular polysaccharide and a carrier protein.

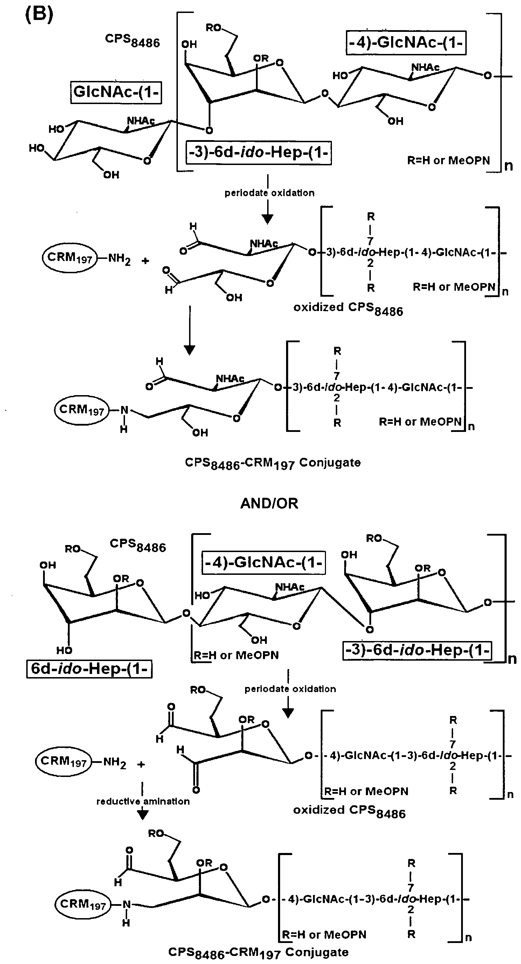

The CPS₈₄₈₆-CRM₁₉₇ glycoconjugates were synthesized by covalent attachment of the CG8486 CPS to CRM₁₉₇ by reductive amination (Figure 5B). The CG8486 CPS to CRM₁₉₇ ratio used here was 2:1 by weight. Here, the non-reducing monosaccharide of CG8486 CPS was oxidized by periodate to yield aldehyde functionalities at the non-reducing end, which served as the attachment point to CRM₁₉₇. Periodate did not oxidize the inner-regions or the reducing-end of these CPS because the lack of available vicinal hydroxyls, and the occupied reducing-ends (by the lipid anchor). Thus, only the non-reducing terminus was oxidized and the structural integrity of the CG CPS remained intact. The oxidized CG8486 CPS was analyzed by NMR, MALDI-TOF-MS and by GC-MS of the alditol acetate derivatives. The characterization of a tri-*O*-acetyl 1-[²H₁]glycerol unit in the oxidized CPS was of particular interest, in that it afforded evidence that oxidation at the non-reducing end had occurred. The MALDI-TOF-MS spectra of the glycoconjugate yielded a broad *m*/*z* ions that, on average, ranged from 70000 to 80000 Da for CPS₈₄₈₆-CRM₁₉₇, which implied that each CRM₁₉₇ was on average carrying up to 5 CPS₈₄₈₆. It is also possible that higher molecular weight CPS₈₄₈₆-CRM₁₉₇ conjugates may be present that could not be detected by MALDI-TOF-MS. Reassurance that glycoconjugates did not contain any detectable amounts of free CPS or CRM₁₉₇ was also observed in the MS spectra and/or or gel-electrophoresis, in that neither free CPS nor CRM₁₉₇ was detected in the MALDI-TOF-MS or SDS-PAGE analyses of the glycoconjugate synthesized here.

### Example 2: Immunogenicity of the CG8486 capsule conjugate vaccine (CPS₈₄₈₆-CRM₁₉₇₎

Mice were immunized with 3 doses of either 1, 5 and 25 micrograms of the capsule conjugate from CG8486 subcutaneously at four-week intervals. Blood samples were collected immediately before each vaccination and at 4, 8, 12 and 14 weeks after the third vaccination. The CPS₈₄₈₆ IgG titers were determined by ELISA. Animals receiving PBS showed baseline levels of CPS₈₄₈₆-specific IgG titer (geometric endpoint titer 3.4 ± 0.40). The titers of immunized animals are shown in Table 2. Immunization with 1 µg of CG8486 capsule conjugate vaccine failed to induce CPS₈₄₈₆-specific IgG. In contrast, animals immunized with 5 and 25 µg of vaccine had similar high levels of antigen-specific serum IgG after two doses. Delivery of the third dose of 5 µg of vaccine further enhanced IgG levels, but vaccination with the third dose of 25 µg of vaccine did not. The peak levels of IgG titers in the groups that received 5 µg and 25 µg of the CG8486 capsule conjugate vaccine are significantly higher than those that received either 1 µg of the vaccine or PBS, but are not significantly different than each other. After completion of the immunization series, ≥90% of the animals met the definition of responders in the groups receiving the two higher doses. CPS₈₄₈₆-specific IgG levels remained elevated for at least 14 weeks after the third dose.

**Table2: Kinetics of CPS₈₄₈₆-specific serum IgG after vaccination with CPS₈₄₈₆-CRM₁₉₇.**

| Vaccine dose (µg) | After vaccination | | Week after vaccination 3 | | | |
|---|---|---|---|---|---|---|
| | 1 | 2 | 4 | 8 | 12 | 14 |
| 1 | 3.36±0.44 (0) | 3.57±0.77 (10) | 3.87±1.29 (20) | 3.98±1.58 (20) | 3.81±1.18 (20) | 3.54±0.90 (10) |
| 5 | 3.42±0.64 (10) | 6.42±2.00 (50) | 9.85±1.08 (100) | 9.65±1.13 (100) | 9.23±0.78 (100) | 7.55±1.21 (100) |
| 25 | 3.57±0.75 (10) | 8.34±2.28 (80) | 10.15±1.50 (100) | 9.13±1.86 (90) | 9.55±2.03 (90) | 8.85±1.84 (100) |

| | | | | | | |
|---|---|---|---|---|---|---|
| *Responders were animals showing an endpoint titer of ≥1:100 (logₑ ≥4.6; equivalent to mean+3SDof PBS recipients). The 1 microgram doses no significant change from the base line at any time (p > 0.05); for 5 µg doses there was a significant increase after dose 2 (p <0.001), which further increased after dose 3 (p >0.001); for 25 µg doses there was a significant increase after dose 2 (p <0.001), which did not increase after dose 3 (p < 0.05). For both the 5 and 25 µg doses the titers remained significantly higher post dose 1 (p<0.001). No significant difference (p > 0.05) between IgG levels of the 5 and 25 µg doses was seen at any time. | | | | | | |

### Example 3: Protective efficacy of CPS₈₄₈₆-CRM₁₉₇ in an intranasal mouse model of C. jejuni infection.

To determine the ability of the CG8486 capsule conjugate vaccine to protect against homologous challenge in the mouse intranasal model of infection (FIG. 6), animals immunized with 5 or 25 µg of the CG8486 capsule conjugate vaccine or PBS at 4-week intervals were challenged intranasally with *C. jejuni* strain CG8486. The illness indices were calculated as described in the figure legend. Vaccinated animals never reached the same level of disease severity as that seen with control animals. On days 1 and 1.5 after challenge, animals immunized with 25 µg showed significantly lower sickness than controls or the recipients of 5 µg (p<0.05), although severity of sickness increased until day 3. Three days after challenge animals immunized with either dose of the vaccine showed significantly lower sickness indices than controls (p=0.05). The mean sickness index returned to normal by day 4.5 (25 µg) or 5.5 (5 µg). In contrast, 50% of control animals remained sick for the 6-day observation period.

### Example 4: Prophetic example of induction of immunity to capsule in humans using BH0142 Capsular Polysaccharide (CPS)

An aspect of this invention is the ability of one or more related isolated disaccharide polymers found in *C*. *jejuni* capsules to induce a vigorous and efficacious immune response in humans but not induction of contraindicating Guillain Barre Syndrome. For each vaccine formulation containing capsules from a single or mixtures of *C*. *jejuni* strains, a limited amount of experimentation is required to ascertain the optimal effective dose ranges. However, a prophetic method for the induction of anti-*C*. *jejuni* medicated diarrheal protective immunity contains the following steps:
a. priming is by administration of an immunogenic formulation containing isolated *C*. *jejuni* capsular polysaccharide composed of disaccharide repeats with the general formula

   →4)-[P→3]-alpha-D-Gal-(1→3)-[P→2/7]-6d-alpha-D-ido-Hep-(1→

   or

   →4)-[P→3]-alpha-D-Gal-(1→3)-[P→2/7]-L-glycero-alpha-D-ido-Hep-(1→

   where P represents non-stoichiometric O-methyl phosphoramidate. Alternatively the immunogenic formulation can contain a mixture of both disaccharide structures. In a preferred embodiment, the isolated disaccharides are conjugated to a carrier molecule. The immunogenic formulation can be administered orally, nasally, subcutaneously, intradermally, transdermally, transcutaneously intramuscularly, or rectally. Depending on the route of administration, the vaccine formulation can be administered with or without any of a number of adjuvants, including but not limited to LTR 192G, Aluminum hydroxide, RC529E, QS21, E294, oligodeoxynucleotides (ODN), CpG-containing oligodeoxynucleotides, aluminum phosphate, MPL^{®} (GlaxoSmithKline, Middlesex, UK) or combinations of these or other potential adjuvants. The range of a unit dose of immunogen is 0.1 µg to 10 mg of immunogen in a range of buffer solutions.
b. Subsequent to a priming dose, 1 to 4 boosting doses can also be administered with unit dose range of 0.1 µg to 10 mg of immunogen in a buffered aqueous solution with or without adjuvant.

### REFERENCES

1. Allos, B. M. 1997. Association between Campylobacter infection and Guillain Barre Syndrome. J. Infect. Dis. 176(Suppl 2):S125-128.
2. Anderson, P. W. 1983. Antibody responses to Haemophilus influenzae type b and diptheria toxin induced by conjugates of oligosaccharides of the type b capsule with the non toxic protein CRM197. Infect. Immun. 39:233-238.
3. Aspinall, G. O., S. Fujimoto, A. G. MacDonald, H. Pang, L.A. Kuryjanczyk and J. L. Penner. 1994a. Lipopolysaccharides from Campylobacter jejuni associated with Guillain Barre Syndrome patients mimic human gangliosides in structure. Infect. Immun. 62:2122-2125.
4. Aspinall, G. O., McDonald, A. G., and Pang, H. 1992. Structures of the O chain from lipopolysaccharides of Campylobacter jejuni serotypes O:23 and O:36. Carbohyr.Res. 231:13-20.
5. Aspinall, G. O., A. G. MacDonald, H. Pang, L. A. Kurjanczyk, and J. L. Penner. 1994b. Lipopolysaccharides of Campylobacter jejuni serotype O:19: structures of core oligosaccharide regions from the serostrain and two bacterial isolates from patients with Guillain Barre Syndrome. Biochem. 33:241-249.
6. Aspinall, G. O., A. G. MacDonald, T. S. Raju, H. Pang, L. A. Kurjanczyk, J.L.Penner and A. P. Moran. 1993. Chemical structure of the core region of Campylobacter jejuni serotype O:2 lipopolysaccharide. Eur. J. Biochem. 213:1029-1037.
7. Aspinall, G. O., C. M. Lynch, H. Pang, R. T. Shaver, A. P. Moran. 1995. Chemical structures of the core region of Campylobacter jejuni O:3 lipopolysaccharide and an associated polysaccharide. Eur. J. Biochem. 231(3): 570-578.
8. Baqar, S., Bourgeois, A. L., Applebee, L A., Mourad, A. S., Kleinosky, M. T., Mohran, Z., and J. R. Murphy. 1996. Murine intranasal challenge model for the study of Campylobacter pathogenesis and immunity. Infect. Immun. 64:4933-4939.
9. Chu, C., Schneerson, R., Robbins, J. B. and Rastogi, S. C. 1983. Further studies on the immunogenicity of Haemophilus influenzae type b and pneumoccocal type 6A polysaccharide-protein conjugates. Infect. Immun. 40:245-256.
10. Cover, T. L. and M. J. Blaser. 1999. The pathobiology of Campylobacter infections in humans. Ann. Rev. Med. 40:269-185.
11. Goon, S., J. F. Kelly, S. M. Logan, C. P. Ewing, P. Guerry. 2003. Pseudaminic acid, the major modification of Campylobacter flagellin, is synthesized via the Cj1293 gene. Mol. Microbiol. 50(2):659-671.
12. Hanniffy, O.M., A.S. Shashkov, A.P. Moran, M.M. Prendergast, S.N. Senchenkova, Y.A. Knirel, and A.V. Savage. 1999. Chemical structure of a polysaccharide from Campylobacter jejuni 176.83 (serotype O:41) containing only furanose sugars. Carbohydr. Res. 319:124-132.
13. Jennings, H. J. Capsular polysaccharides as vaccine candidates. 1990. Curr. Top. Microbiol. Immunol. 150:97-127.
14. Jennings, H. J. and Lugowshi, C. 1981. Immunochemistry of groups A, B and C meningococcal polysaccharides-tetanus toxoid conjugates. J. Immunol. 127:1011-1018.
15. Jennings, H. J., C. W. Lugowski, F. E. Ashton, J. A. Ryan. 1983. The structure of the capsular polysaccharide obtained from a new serogroup (L) of Neisseria meningitidis. Carbohydr. Res. 112(1)::105-111.
16. Karlyshev, A. V., Linton, D., Gregson, N. A., Lastovica, A. J. and Wren, B. W. 2000. Genetic and biochemical evidence of a Campylobacter jejuni capsular polysaccharide that accounts for Penner serotype specificity. Mol. Microbiol. 35:529-541.
17. Marburg, S., Jorn, D., Tolman, R. L., Arison, B., McCauley, J., Kniskern, P. J., Hagopian, A. and Vella, P. P. 1986. Biomolecular chemistry of macromolecules: synthesis of bacterial polysaccharide conjugates with Neisseria meningiditis membrane protein. J. Am. Chem. Soc. 108:5282-5287.
18. McNally, D.J., H.C. Jarrell, J.Li, N.H. Khieu, E. Vinogradov, C.M. Szymanski, and J.R. Brisson. 2005. The HS:1 serostrain of Campylobacter jejuni has a complex teichoic acid-like capsular polysaccharide with nonstochiometric fructofuranose branches and O-methyl phosphoramidite groups. FEBS J. 272:4407-4422.
19. Moran, A. P., B. J. Appelmelk, and G. O. Aspinall. 1996. Molecular mimicry of host structures by lipopolysaccharides of Campylobacter and Helicobacter spp.: implications in pathogenesis. J. Endotox. Res. 3(6):521-531.
20. Moran, A. P. and J. L. Penner. 1999. Serotyping of Campylobacter jejuni based on heat-stable antigens: relevance, molecular basis and implications in pathogenesis. J. Appl. Microbiol. 86:361-377.
21. Muldoon, J., A. S. Shashkov, A. P. Moran, J. A. Ferris, S. N. Senchenkova, and A. V. Savage. 2002. Structures of two polysaccharides of Campylobacter jejuni 81116. Carbo. Res. 337:2223-2229.
22. Parkhill, J., B. W. Wren, K. Mungall, J. M. Ketley, C. Churcher, D. Basham, T. Chillingworth, R. M. Davies, T. Feltwell, S. Holroyd, K. Jagels, A. V. Karlyshev, S. Moule, M. J. Pallen, C. W. Penn, M. A. Quail, M. A. Rajandream, K. M. Rutherford, A. H. M. van Vliet, S. Whitehead, and B. G. Barrell. 2000. The genome sequence of the food-bornne pathogen Campylobacter jejuni reveals hypervariable tracts. Nature 403:665-668.
23. Power, P. M. and Jennings, M. P. 2003. The genetics of glycosylation in gram negative bacteria. FEMS Microbiol. Lett. 218:211-222.
24. Russell, R.G., M.J. Blaser, J.I. Sarmiento and J. Fox. 1989. Experimental Camplobacter jejuni infectin in Macaca nemestrina. Infect. Immun. 57:1438-1444.
25. Russell, R.G., M. O'Donnoghue, D.C. Jr. Blake, J. Zulty and L.J. DeTolla. 1993. Early colonic damage and invasion of Camplylobacter jejuni in experimentally challenged infant Macaca mulatta. J. Infect. Dis. 168:210-215.
26. Salloway, S., L. A. Mermel, M. Seamans, G. O. Aspinall, J. E. Nam Shin, L. A. Kurjanczyk, and J. L. Penner. 1996. Miller Fisher Syndrome associated with Campylobacter jejuni bearing lipopolysaccharide molecules that mimic human ganglioside GD3. Infect. Immun. 64:2945-2949.
27. Schneerson, R., Barrera, O., Sutton, A. and Robbins, J. B. 1980. Preparation, characterization and immunogenicity of Haemophilus influenzae type b polysaccharide protein conjugates. J. Exp. Med. 152:361-376.
28. St. Michael, F., C. M. Szymanski, J. Li, K. H. Chan, N. H.Khieu, S. Larocque, W. W. Wakarchuk, J.-R. Brisson, and M. A. Monteiro. 2002. The structures of the lipooligosaccharide and capsule polysaccharide of Campylobacter jejuni genome sequenced strain NCTC 11168. Eur. J. Biochem. 269:5119-5136.
29. Szymanski, C.M., Yao, R., Ewing, C.P., Trust, T.J., and Guerry, P. 1999. Evidence for a system of general protein glycosylation in Campylobacter jejuni. Mol Microbiol 32:1022-1030.
30. Szymanski, C. M., Logan, S. M., Linton, D., and Wren, B. W. 2003. Campylobacter-a tale of two protein glycosylation systems. Trends Microbiol. 11:233-238.
31. Tauxe, R. V. 1992. Epidemiology of Campylobacter jejuni infections in the United States and other industrialized nations. In Campylobacter jejuni: Current status and future trends (Edited by Nachamkin I., Blaser M. J. and Tompkins L. S.), p. 9. American Society for Microbiology, Washington, D.C..
32. Taylor, D. N. 1992. Campylobacter infections in developing countries. In Campylobacter jejuni: Current status and future trends (Edited by Nachamkin I., Blaser M. J. and Tompkins L. S.), p. 20. American Society for Microbiology, Washington, D.C..
33. Thibault, P., Logan, S. M., Kelly, J.F., Brisson, J.-R., Ewing, C. P., Trust, T. J., and Guerry, P. 2001. Identification of the carbohydrate moieties and glycosylation motifs in Campylobacter jejuni flagellin. J Biol Chem 276: 34862-34870.
34. Young, N. M., Brisson, J.-R., Kelly, J., Watson, D. C., Tessier, L., Lanthier, P. H., Jarrell, H. C., Cadotte, N., St. Michael, F., Aberg, E., and Szymanski, C. M. 2002. Structure of the N linked glycan present on multiple glycoproteins in the gram negative bacterium, Campylobacter jejuni. J. Biol. Chem. 277:42530-42539.
35. Aspinall, G. O. Lipopolysaccharide and associated carbohydrate polymers from Campylobacter jejuni and Helicobacter pylori. 1998. Carbohydr. Eur. 2 1: 24-29.
36. Chen, Y-H., Poly, F, Pakulski, Z., Guerry, P., and Monteiro, M. A. 2008. The chemical structure and genetic locus of Campylobacter jejuni CG8486 (serotype HS:4) capsular polysaccharide: The identification of 6-deoxy-D-ido-heptopyranose. Carbohydr. Res. 343: 1034-1040.
37. Baqar, S., A. L. Bourgeois, L. A. Applebee, A. S. Mourad, M. T. Kleinosky, Z. Mohran, and J. R. Murphy. 1996. Murine intranasal challenge model for the study of Campylobacter pathogenesis and immunity. Infect. Immun. 64::4933-4939.

## Claims

1. An immunogenic composition, composed of an isolated polymer, wherein said polymer contains repeat units of a disaccharide having the formula
→4)-[P→3]-alpha-D-Gal-(1→3)-[P→2/7]-6d-alpha-D-ido-Hep-(1→,
wherein P is non-stoichiometic O-methyl phosphoramidate.

2. The immunogenic composition according to Claim 1, **characterized in that** said repeating disaccharide is conjugated to a carrier molecule.

3. The immunogenic composition according the claim 1, **characterized in that** said isolated polymer further comprises 3-hydroxypropanoyl.

4. The immunogenic composition according to claim 1, **characterized in that** said isolated polymer contains 2-100 repeat units of said disaccharide.

5. The immunogenic composition according to one of claims 1-4 for use in the prevention of *Campylobacter jejuni* infections.

6. The immunogenic composition for use according to claim 5, **characterized in that** said isolated polymer is conjugated to a carrier molecule.

7. The immunogenic composition for use according to claim 6, **characterized in that** the carrier molecule is CRM₁₉₇.

8. The immunogenic composition for use according to claim 5, wherein said immunogenic composition contains 2-100 repeat units of said disaccharide.

## Patentansprüche

1. Immunogene Zusammensetzung, zusammengesetzt aus einem isolierten Polymer, wobei das Polymer Wiederholungseinheiten eines Disaccharids mit der Formel
→4)-[P→3]-alpha-D-Gal-(1→3)-[P→2/7]-6d-alpha-D-ido-Hep-(1→,
enthält, wobei P nichtstöchiometrisches O-Methylphosphoramidat ist.

2. Immunogene Zusammensetzung nach Anspruch 1, **dadurch gekennzeichnet, dass** das Wiederholungs-Disaccharid an ein Trägermolekül konjugiert ist.

3. Immunogene Zusammensetzung nach Anspruch 1, **dadurch gekennzeichnet, dass** das isolierte Polymer ferner 3-Hydroxypropanol umfasst.

4. Immunogene Zusammensetzung nach Anspruch 1, **dadurch gekennzeichnet, dass** das isolierte Polymer 2 bis 100 Wiederholungseinheiten des Disaccharids enthält.

5. Immunogene Zusammensetzung nach einem von Anspruch 1 bis 4 zur Benutzung zur Vorbeugung gegen *Campylobacter-jejuni-*Infektionen.

6. Immunogene Zusammensetzung zur Benutzung nach Anspruch 5, **dadurch gekennzeichnet, dass** das isolierte Polymer an ein Trägermolekül konjugiert ist.

7. Immunogene Zusammensetzung zur Benutzung nach Anspruch 6, **dadurch gekennzeichnet, dass** das Trägermolekül CRM₁₉₇ ist.

8. Immunogene Zusammensetzung zur Benutzung nach Anspruch 5, wobei die immunogene Zusammensetzung 2 bis 100 Wiederholungseinheiten des Disaccharids enthält.

## Revendications

1. Composition immunogène, composée d'un polymère isolé, dans laquelle ledit polymère contient des unités répétées d'un disaccharide répondant à la formule
→4)-[P→3]-alpha-D-Gal-(1→3)-[P→2/7]-6d-alpha-D-ido-Hep-(1→
dans laquelle P est un O-méthyl phosphoramidate non stoechiométrique.

2. Composition immunogène selon la revendication 1, **caractérisée en ce que** ledit disaccharide récurrent est conjugué à une molécule porteuse.

3. Composition immunogène selon la revendication 1, **caractérisée en ce que** ledit polymère isolé comprend en outre un groupe 3-hydroxypropanoyl.

4. Composition immunogène selon la revendication 1, **caractérisée en ce que** ledit polymère isolé contient 2 à 100 unités de répétition dudit disaccharide.

5. Composition immunogène selon l'une quelconque des revendications 1 à 4, pour son utilisation dans la prévention d'infections à *Campylobacter jejuni.*

6. Composition immunogène pour son utilisation selon la revendication 5, **caractérisée en ce que** ledit polymère isolé est conjugué à une molécule porteuse.

7. Composition immunogène pour son utilisation selon la revendication 6, **caractérisé en ce que** la molécule porteuse est CRM₁₉₇.

8. Composition immunogène pour son utilisation selon la revendication 5, dans laquelle ladite composition immunogène contient de 2 à 100 unités de répétition dudit disaccharide.
